# EUROPÄISCHE PATENTSCHRIFT

(11) **EP 0 928 164 B1**
(45) Veröffentlichungstag und Bekanntmachung des Hinweises auf die Patenterteilung: **27.08.2003**
(21) Anmeldenummer: 97929225.7
(22) Anmeldetag: 19.06.1997
(51) Int. Cl.: A61B 17/16

(54) **KNOCHENFRÄSER**
BONE CUTTER
FRAISE POUR OS

(30) Priorität: 26.08.1996 DE 19634484
(43) Veröffentlichungstag der Anmeldung: 14.07.1999
(73) Patentinhaber: PLUS ENDOPROTHETIK AG, 6343 Rotkreuz (CH)
(72) Erfinder: ECKHARDT, Harald, D-83071 Stephanskirchen (DE); KRAUSE, Gero, D-83071 Stephanskirchen (DE)
(74) Vertreter: Popp, Eugen, Dr.
(86) Internationale Anmeldenummer: EP9703211
(87) Internationale Veröffentlichungsnummer: WO98008444

(56) Entgegenhaltungen:
- DE-A- 2 748 452
- DE-A- 3 007 036
- US-A- 4 976 740
- PATENT ABSTRACTS OF JAPAN vol. 14, no. 87 (M-0937), 19.Februar 1990 & JP 01 301007 A (YOKOHAMA RUBBER), 5.Dezember 1989,

## Beschreibung

Die Erfindung betrifft einen Knochenfräser zur genauen Präparation von Knochen gemäß dem Oberbegriff des Anspruchs 1.

Aus der DE-OS 27 48 452 ist ein Knochenfräser zum Rundfräsen eines Hüftgelenkkopfes bekannt, der mit einem zentralen Loch versehen ist, in das ein Zentrierstift eingeführt wird. Der Fräskopf ist mit einer Antriebswelle verbunden, die zumindest im fräskopfnahen Bereich als Hohlwelle ausgebildet ist.

Ferner ist ein Knochenfräser bekannt, wie er in Fig. 4 dargestellt ist. Ein solcher Knochenfräser wird zur paßgenauen Präparation von Knochen, speziell von Röhrenknochen verwendet. Insbesondere bei der zementfreien Implantation von Prothesen ist es wichtig, ein möglichst paßgenaues Knochenbett zur Aufnahme der Prothese zu schaffen, um zum einen eine möglichst große Kontaktfläche zum schnellen Anwachsen des Knochens sicherzustellen, und zum anderen Mikrobewegungen auszuschalten. Da oszillierende Sägen zu wenig genau sind, werden rotierende Instrumente bevorzugt.

Der in Fig. 4 dargestellte Knochenfräser 10a ist ein geführter Stirnfräser. Er besteht aus einem zylindrischen Fräskopf 12a mit an der Frontseite angeordneten Schneidzähnen. Auf dieser Seite ist auch ein Führungsbolzen 30 ausgebildet. Auf der Rückseite des Fräskopfes 12a ist eine Antriebswelle 14' befestigt. Bei der Verwendung dieses Knochenfräsers 10a wird der Führungsbolzen 30 in eine in den Knochen eingebrachte Führungsbohrung gesteckt, so daß solchermaßen geführt eine richtungsstabile Bearbeitung sichergestellt ist. Durch Ausüben von Druck (vgl. Pfeile 26 in Fig. 4) auf die Antriebswelle 14' in Richtung des Führungsbolzens 30 kommen die an der Frontseite angeordneten Schneidezähne mit dem Knochen in Kontakt und fräsen eine rotationssymmetrische Fläche in die Tiefe.

Problematisch bei beiden oben genannten Knochenfräsern ist jeweils die Antriebswelle, die aus dem Operationsfeld herausragt und mit einem Antriebsaggregat verbunden werden muß. Ist das Operationsfeld klein und muß der Zugang aus anatomischen oder chirurgischen Gründen eng gestaltet werden, kann es vorkommen, daß beim Niederbringen der Fräsung die Antriebswelle durch Vorsprünge in dem Operationsfeld aus der Richtung gedrängt wird, was zu einer richtungsinstabilen oder richtungsfalschen Fräsfläche führt. Das Ergebnis ist ein ungenaues Knochenbett, sowohl bezüglich der Kontaktfläche als auch bezüglich der Positionierung einer Prothese.

Aufgabe der Erfindung ist es, einen Knochenfräser zu schaffen, mit dem auch bei kleinem Operationsfeld die Ausbildung eines möglichst paßgenauen Knochenbetts ohne weiteres möglich ist.

Diese Aufgabe wird durch die im Anspruch 1 genannten Merkmale gelöst.

Gemäß der in Anspruch 1 angegebenen Erfindung dient die Antriebswelle gleichzeitig als Führungsorgan und wird durch eine in den Knochen eingebrachte durchgehende Führungsbohrung hindurch geführt. Da die Zahnung antriebswellenseitig am Fräskopf angeordnet ist, wird - im Gegensatz zu herkömmlichen Knochenfräsern - nicht mehr auf Druck, sondern auf Zug gearbeitet.

Beim Einsatz des Knochenfräsers verläuft die Antriebswelle vom Fräskopf in Richtung des Knochen und durch die in diesem eingebrachte Führungsbohrung hindurch. Damit ragt die Antriebswelle nicht mehr aus dem Operationsfeld heraus und kann auch nicht mehr durch irgendwelche in das Operationsfeld hineinragenden Gegenstände aus der Richtung gedrängt werden. Die Ausbildung eines sehr paßgenauen Knochenbetts ist somit sichergestellt.

Vorzugsweis ist die Antriebswelle flexibel ausgestaltet. Die Verwendung einer flexiblen Antriebswelle wird erst dadurch ermöglicht, daß nicht mehr auf Druck, sondern auf Zug gearbeitet wird.

Gemäß einer weiteren vorteilhaften Ausführungsform ist in der in den Knochen eingebrachten Führungsbohrung ein Führungsrohr eingebracht. Dadurch reibt die Antriebswelle nicht am Knochen oder an den anliegenden Weichteilen. Das Führungsrohr kann von dem dem Operationsfeld gegenüberliegenden Ende der Bohrung her in diese eingeschoben werden. Beim Niederbringen der Fräsung schiebt der Fräskopf das Führungsrohr vor sich her, ohne es anzufräsen.

Da die Antriebswelle durch den Knochen vollständig hindurchgeführt werden muß und sie auf einer Seite mit dem Fräskopf, auf der anderen Seite mit einem Antriebsaggregat verbunden ist, muß zumindest eine dieser Verbindungen lösbar ausgebildet sein. Die lösbare Verbindung ist vorzugsweise am Übergang Antriebswelle-Fräskopf oder am Übergang Antriebswelle-Antriebsaggregat vorgesehen.

Im ersteren Fall wird vor dem Fräsvorgang die Antriebswelle vom gegenüberliegenden Ende der Führungsbohrung her in Richtung Operationsfeld eingebracht und mit dem Fräskopf verbunden. Im zweiten Fall wird die Antriebswelle vom Operationsfeld her durch die Führungsbohrung gesteckt und beim Austritt aus dem Knochen auf der gegenüberliegenden Seite mit dem Antriebsaggregat verbunden.

Der Fräskopf kann verschieden gestaltet werden, sollte jedoch rotationssymmetrisch ausgebildet sein. Für eine zylindrische Ausfräsung wird ein Fräskopf gewählt, dessen Zahnung in einer Ebene angeordnet ist. Das erzeugt eine zur Antriebswelle senkrecht stehende, ebene Schnittfläche. Alternativ kann auch ein Fräskopf verwendet werden, der eine Schneidfläche aufweist, die konkav, konvex oder als Kombination solcher Flächen ausgebildet ist. Damit lassen sich sphärische, konische, zylindrische, polygone, wellige oder sonstige beliebige, rotationssymmetrische Formoberflächen fräsen.

Eine bevorzugte Ausführungsform ist dadurch gekennzeichnet, daß die schneidende Fläche durch einen nichtschneidenden Schutzkragen verlängert wird. Dadurch können allfällige Weichteile von der Fräszone ferngehalten werden.

Die Zahnung des Fräskopfes kann konventionell ausgebildet, d.h. gefräst oder gehauen sein. Besonders vorteilhaft ist jedoch die Verwendung einer raffelartigen Zahnung, wobei zusätzlich spanabführende Löcher im Fräskopf vorgesehen sind. Damit ist es möglich, die Knochenspäne aus dem Fräsbereich abzuführen.

Eine besondere Kombination mit der letztgenannten Ausführungsform einer Zahnung ergibt sich dann, wenn auf der Rückseite des Fräskopfes eine Auffangvorrichtung, insbesondere ein Sammelbehältnis angeordnet ist. Nutzbringend wäre beispielsweise die Verwendung eines Körbchens oder schüsselartigen Behältnisses, in welchem die anfallenden Knochenspäne gesammelt werden. Dadurch kann nicht nur eine Pollution des Operationsfeldes durch solche Späne verhindert und die Gefahr eines induzierten, unerwünschten, ektopischen Knochenwachstums eingedämmt werden. Es ist auch möglich, die im Behältnis gesammelten Späne zu wertvollem, autologem Spongiosabrei weiterzuverwenden.

Die Erfindung wird nachfolgend, auch in Hinblick auf weitere Vorteile und Merkmale, anhand von Ausführungsbeispielen und mit Bezug auf die beiliegenden Zeichnungen näher erläutert. Die Zeichnungen zeigen in
- Fig. 1: eine schematische Seitenansicht eines Ausführungsbeispiels des erfindungsgemäßen Knochenfräsers,
- Fig. 2: eine Schnittdarstellung einer weiteren Ausführungsform des erfindungsgemäßen Knochenfräsers mit domförmiger Schnittfläche und Schraubverbindung zwischen Fräskopf und Antriebswelle
- Fig. 3: eine schematische, perspektivische Darstellung einer dritten Ausführungsform eines erfindungsgemäßen Knochenfräsers mit raffelartiger Zahnung und Sammelbehältnis und
- Fig. 4: eine schematische Seitenansicht eines Knochenfräsers nach dem Stand der Technik.

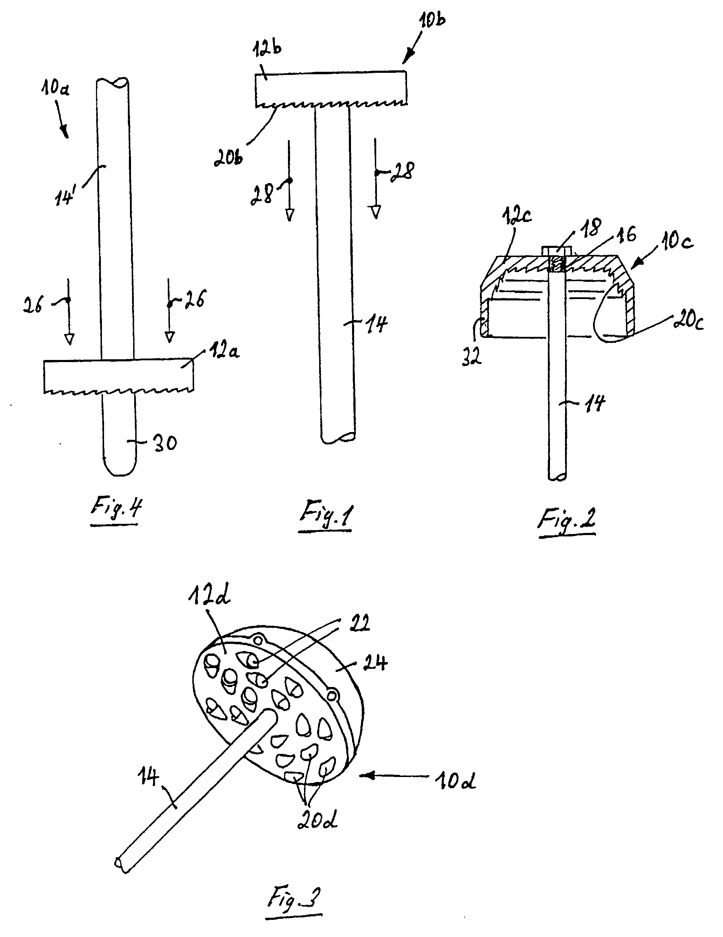

In Fig. 1 ist ein Ausführungsbeispiel eines Knochenfräsers 10b dargestellt, der im wesentlichen aus einem zylinderförmigen Fräskopf 12b und einer Antriebswelle 14 besteht.

Der Fräskopf 12b weist an seiner zur Antriebswelle 14 weisenden Fläche eine Zahnung 20b auf, die - bei vorliegendem Ausführungsbeispiel - gefräst ist. Sie kann auch gehauen oder anderweitig ausgebildet sein. Alle Zähne der Zahnung 20b liegen im wesentlichen in einer Ebene, so daß beim Einsatz eine sich senkrecht zur Antriebswelle 14 erstreckende Schnittfläche erhalten wird.

Die Antriebswelle 14 ist fest mit dem Fräskopf 12b verbunden und dient gleichzeitig als Führungselement, entsprechend dem Führungsbolzen 30 aus Fig. 4. Um den Knochenfräser 10b einsetzen zu können, muß in den Knochen zunächst eine durchgehende Führungsbohrung eingebracht werden. Für eine Formfräsung z.B. eines Oberschenkelkopfes wird durch den Schenkelhals die durchgehende Führungsbohrung eingebracht. Die Antriebswelle 14 wird vom Schenkelkopf her durch die Führungsbohrung gesteckt und beim Austritt aus dem Knochen auf der lateralen Seite mit einem (nicht dargestellten) Antriebsaggregat verbunden.

Da bei dem vorliegenden Knochenfräser 10b Führungsbolzen und Antriebswelle 14 zusammenfallen und die Zahnung 20b nicht auf der Stirnseite, sondern auf der Rückseite angeordnet ist (ähnlich wie die Lamellen bei einem Pilz), arbeitet der Knochenfräser 10b nicht mittels Druck auf die Antriebswelle 14, sondern durch Zug auf dieselbe (vgl. Pfeile 28). Dadurch ist es möglich, auch mit einer flexiblen Antriebswelle 14 zu arbeiten.

Beim Arbeiten mit einer flexiblen Antriebswelle ist es von Vorteil, die Führungsbohrung von lateral mit einem (nicht dargestellten) Führungsrohr auszukleiden, so daß die rotierende Antriebswelle 14 nicht am Knochen oder an den anliegenden Weichteilen reibt. Beim Niederbringen stößt der Fräskopf 12b das Führungsrohr einfach vor sich her, ohne dieses selbst anzufräsen.

Fig. 2 zeigt ein weiteres Ausführungsbeispiels eines Knochenfräsers 10c, wobei die gleichen Bezugsziffern gleichen Teilen entsprechen. Der Knochenfräser 10c unterscheidet sich von demjenigen gemäß Fig. 1 in zweierlei Hinsicht.

Zum einen ist der Fräskopf 12c domförmig ausgebildet, wie in der Schnittdarstellung zu erkennen ist. Auf der der Antriebswelle 14 zugeordneten Seite des Fräskopfes 12c ist eine konkave Schneidfläche mit entsprechender Zahnung 20c vorgesehen. Damit läßt sich eine dieser Fläche entsprechende Formoberfläche fräsen. Um Weichteile von der Schneidzone fern zu halten, ist die Schneidfläche durch einen überstehenden, nichtschneidenden Schutzkragen 32 verlängert.

Zum anderen sind die Antriebswelle 14 und der Fräskopf 12c lösbar miteinander verbunden, und zwar mittels einer Schraube 18. Diese ist durch eine zentrische Bohrung 16 im Fräskopf 12c hindurchgeführt und in ein Innengewinde am fräskopfseitigen Ende der Antriebswelle 14 eingeschraubt. Bei einem solchen Knochenfräser 10c wird vor dem Fräsvorgang die Antriebswelle 14 lateral vom Ende der Führungsbohrung her in Richtung des Schenkelkopfes eingebracht und dann mit dem Fräskopf 12c mittels der Schraube 18 verbunden.

Eine dritte Ausführungsform eines Knochenfräsers 10d ist in Fig. 3 dargestellt. Dieser Knochenfräser 10d weist gegenüber den oben genannten Ausführungsbeispielen - insbesondere demjenigen aus Fig. 1 - folgende Besonderheit auf. Die Zahnung 20d auf der antriebsseitigen Fläche des Fräskopfes 12d ist raffelartig ausgebildet und weist sich durch den Fräskopf 12d hindurcherstreckende spanabführende Löcher 22 auf. Über der der Antriebswelle 14 gegenüberliegenden Fläche des Fräskopfes 12d ist ein abnehmbares Sammelbehältnis 24 angeordnet, so daß alle von der raffelförmigen Zahnung 20d abgetrennten und durch die spanabführenden Löcher 22 hindurch transportierten Knochenspäne in diesem Sammelbehältnis 24 aufgefangen werden können. Dadurch wird eine Verschmutzung des Operationsfeldes wirkungsvoll vermieden, wodurch die Gefahr eines induzierten, unerwünschten ektopischen Knochenwachstums eingedämmt ist. Die in dem abnehmbaren Sammelbehältnis 24 eingefangenen Knochenspäne können anschließend zu wertvollem, autologem Spongiosabrei weiterverarbeitet werden.

Insgesamt sind mit den beschriebenen Ausführungsformen Knochenfräser angegeben, mit denen ein paßgenaues Knochenbett beispielsweise zur Aufnahme von Prothesen geschaffen werden kann, ohne daß Elemente des Knochenfräsers aus dem Operationsfeld herausragen und mit Gegenständen im Operationsfeld in Berührung kommen können. Eine richtungsinstabile oder richtungsfalsche Fräsfläche kann wirksam vermieden werden.

Durch die Maßnahmen nach Anspruch 14 erhält man gefräste Knochenflächen, die sich dadurch auszeichnen, daß sie innerhalb kürzester Zeit, z.B. innerhalb von 10 - 14 Tagen vollständig mit mit Hydroxialpatit beschichteten Implantatflächen verwachsen. Damit läßt sich die Einheilung von Implantaten erheblich beschleunigen.

### Bezugszeichenliste

- 10a,b,c,d: Knochenfräser
- 12a,b,c,d: Fräskopf
- 14: Antriebswelle
- 14': Antriebswelle beim Stand der Technik
- 16: Bohrung
- 18: Schraube
- 20b,c,d: Zahnung
- 22: spanabführende Löcher
- 24: Sammelbehältnis
- 26: Pfeil (symbolisiert Druckkraft)
- 28: Pfeil (symbolisiert Zugkraft)
- 30: Führungsbolzen
- 32: Schutzkragen

## Patentansprüche

1. Knochenfräser zur genauen Präparation von Knochen umfassend einen Fräskopf (12b, 12c, 12d) und eine mit dem Fräskopf (12b, 12c, 12d) verbundene Antriebswelle (14),
dadurch gekenzeichnet,
daß am Fräskopf (12b, 12c, 12d) antriebswellenseitig eine Zahnung (20b, 20c, 20d) angeordnet ist, und daß die Antriebswelle (14) gleichzeitig als im Knochen geführtes Führungsorgan dient.

2. Knochenfräser nach Anspruch 1,
**dadurch gekennzeichnet,**
**daß** die Antriebswelle (14) flexibel ausgebildet ist.

3. Knochenfräser nach Anspruch 1 oder 2,
**dadurch gekennzeichnet,**
**daß** ein Führungsrohr vorgesehen ist, welches im Knochen plazierbar ist und durch das die Antriebswelle (14) drehbar gelagert hindurchführbar ist.

4. Knochenfräser nach einem der Ansprüche 1 bis 3,
**dadurch gekennzeichnet,**
**daß** die Antriebswelle (14) mit dem Fräskopf (12c) lösbar verbunden ist.

5. Knochenfräser nach einem der Ansprüche 1 bis 3,
**dadurch gekennzeichnet,**
**daß** die Antriebswelle (14) mit einem Antriebsaggregat lösbar verbunden ist.

6. Knochenfräser nach einem der Ansprüche 1 bis 5,
**dadurch gekennzeichnet,**
**daß** der Fräskopf (12b, 12c, 12d) rotationssymmetrisch ausgebildet ist.

7. Knochenfräser nach Anspruch 6,
**dadurch gekennzeichnet,**
**daß** der Fräskopf (12b, 12d) eine im wesentlichen plan ausgebildete Schneidfläche aufweist.

8. Knochenfräser nach Anspruch 6,
**dadurch gekennzeichnet,**
**daß** der Fräskopf (12c) eine Schneidfläche aufweist, die konkav, konvex oder als Kombination von konkaven, konvexen oder planen Flächen ausgebildet ist.

9. Knochenfräser nach einem der Ansprüche 1 bis 8,
**dadurch gekennzeichnet,**
**daß** die Schneidfläche des Fräskopfes (12b, 12c, 12d) durch einen nichtschneidenden, über den Fräskopf vorstehenden Schutzkragen (32) verlängert ist.

10. Knochenfräser nach einem der Ansprüche 1 bis 9,
**dadurch gekennzeichnet,**
**daß** die Zahnung (20b, 20c) des Fräskopfes (12b, 12c) gefräst oder gehauen ist.

11. Knochenfräser nach einem der Ansprüche 1 bis 9,
**dadurch gekennzeichnet,**
**daß** die Zahnung (20d) des Fräskopfes (12d) raffelartig mit spanabführenden Löchern (22) ausgebildet ist.

12. Knochenfräser nach Anspruch 11,
**dadurch gekennzeichnet,**
**daß** eine Auffangvorrichtung (24) am Fräskopf (12d) lösbar angeordnet ist, um die durch die spanabführenden Löcher (22) hindurchtretenden Knochenspäne aufzusammeln oder abzuführen.

13. Knochenfräser nach Anspruch 12,
**dadurch gekennzeichnet,**
**daß** die Auffangvorrichtung als Sammelbehältnis, insbesondere in Form eines Körbchens (24), ausgebildet ist.

14. Knochenfräser nach einem der Ansprüche 1 bis 13,
**dadurch gekennzeichnet,**
**daß** die Schneidflächen der Fräserzahnung (20b, 20c, 20d) mit Hartstoff, insbesondere Diamant, Karbid, Nitrid o. dgl. beschichtet sind.

## Claims

1. A milling cutter intended for bones, for precisely dissecting bones, comprising a cutter head (12b, 12c, 12d) and a driving shaft (14) connected to said cutter head (12b, 12c, 12d),
**characterized in that**
a toothing (20b, 20c, 20d) is disposed on the cutter head (12b, 12c, 12d) on the driving shaft side, and said driving shaft (14) serves simultaneously as a guiding element to be guided in the bone.

2. The milling cutter according to claim 1,
**characterized in that**
said driving shaft (14) is formed to be flexible.

3. The milling cutter according to claim 1 or 2,
**characterized in that**
a guiding tube is provided which may be placed in the bone, and through which the driving shaft (14) being rotatably mounted may be guided.

4. The milling cutter according to any one of claims 1 through 3,
**characterized in that**
said driving shaft (14) is releasably connected to the cutter head (12c).

5. The milling cutter according to any one of claims 1 through 3,
**characterized in that**
said driving shaft (14) is releasably connected to a drive assembly.

6. The milling cutter according to any one of claims 1 through 5,
**characterized in that**
said cutter head (12b, 12c, 12d) is formed to be rotationally symmetrical.

7. The milling cutter according to claim 6,
**characterized in that**
said cutter head (12b, 12d) comprises a cutting surface formed to be essentially planar.

8. The milling cutter according to claim 6,
**characterized in that**
said cutter head (12c) comprises a cutting surface that is formed to be concave, convex or as a combination of concave, convex or planar surfaces.

9. The milling cutter according to any one of claims 1 through 8,
**characterized in that**
said cutting surface of said cutter head (12b, 12c, 12d) is extended by a non-cutting protective collar (32) projecting beyond said cutter head.

10. The milling cutter according to any one of claims 1 through 9,
**characterized in that**
the toothing (20b, 20c) of the cutter head (12b, 12c) is milled or cut.

11. The milling cutter according to any one of claims 1 through 9,
**characterized in that**
the toothing (20d) of the cutter head (12d) is formed to be grater-like with chip-discharging holes (22).

12. The milling cutter according to claim 11,
**characterized in that**
a collecting device (24) is releasably disposed on the cutter head (12d) for collecting or discharging the bone chips passing through said chip-discharging holes (22).

13. The milling cutter according to claim 12,
**characterized in that**
said collecting device is formed to be a collecting receptacle, in particular in the form of a small basket.

14. The milling cutter according to any one of claims 1 through 13,
**characterized in that**
the cutting surfaces of the cutter toothing (20b, 20c, 20d) are coated with a hard material, in particular diamond, carbide, nitride or similar.

## Revendications

1. Fraise à os pour la préparation précise d'os, comprenant une tête de fraise (12b, 12c, 12d) et un arbre d'entraînement (14) relié à la tête de fraise (12b, 12c, 12d), **caractérisée en ce qu'**une denture (20b, 20c, 20d) est placée sur la tête de fraise (12b, 12c, 12d) du côté de l'arbre d'entraînement, et **en ce que** l'arbre d'entraînement (14) sert simultanément d'organe de guidage guidé dans l'os.

2. Fraise à os selon la revendication 1, **caractérisée en ce que** l'arbre d'entraînement (14) est réalisé flexible.

3. Fraise à os selon la revendication 1 ou 2, **caractérisée en ce qu'**il est prévu un tube de guidage, qui peut être placé dans l'os et à travers lequel l'arbre d'entraînement (14) peut être enfilé de manière rotative.

4. Fraise à os selon l'une des revendications 1 à 3, **caractérisée en ce que** l'arbre d'entraînement (14) est relié de manière amovible à la tête de fraise (12c).

5. Fraise à os selon l'une des revendications 1 à 3, **caractérisée en ce que** l'arbre d'entraînement (14) est relié de manière amovible à un groupe d'entraînement.

6. Fraise à os selon l'une des revendications 1 à 5, **caractérisée en ce que** la tête de fraise (12b, 12c, 12d) est configurée avec une symétrie de rotation.

7. Fraise à os selon la revendication 6, **caractérisée en ce que** la tête de fraise (12b, 12d) présente une surface de coupe sensiblement plane.

8. Fraise à os selon la revendication 6, **caractérisée en ce que** la tête de fraise (12c) présente une surface de coupe qui est réalisée concave, convexe ou sous forme de combinaison de surfaces concaves, convexes ou planes.

9. Fraise à os selon l'une des revendications 1 à 8, **caractérisée en ce que** la surface de coupe de la tête de fraise (12b, 12c, 12d) est prolongée par une collerette protectrice (32) non tranchante et formant saillie au-delà de la tête de fraise.

10. Fraise à os selon l'une des revendications 1 à 9, **caractérisée en ce que** la denture (20b, 20c) de la tête de fraise (12b, 12c) est fraisée ou battue.

11. Fraise à os selon l'une des revendications 1 à 9, **caractérisée en ce que** la denture (20d) de la tête de fraise (12d) est conformée en peigne avec des trous (22) d'évacuation de copeaux.

12. Fraise à os selon la revendication 11, **caractérisée en ce qu'**un dispositif de collecte (24) est placé amovible sur la tête de fraise (12d), afin de collecter ou d'évacuer les copeaux d'os traversant les trous (22) d'évacuation de copeaux.

13. Fraise à os selon la revendication 12, **caractérisée en ce que** le dispositif de collecte est conformé en récipient de collecte, en particulier en forme de petite corbeille (24).

14. Fraise à os selon l'une des revendications 1 à 13, **caractérisée en ce que** les surfaces de coupe de la denture de fraise (20b, 20c, 20d) sont revêtues d'une matière dure, en particulier de diamant, de carbure, de nitrure ou analogue.
